Europäisches Patentamt

(19) European Patent Office

Office européen des brevets

(11) Publication number: **0 134 650**
**B1**

(12) **EUROPEAN PATENT SPECIFICATION**

(45) Date of publication of patent specification: **27.01.88**

(21) Application number: **84304571.7**

(22) Date of filing: **04.07.84**

(51) Int. Cl.[4]: **C 07 C 53/08,** C 07 C 51/48, C 07 C 51/44

(54) **High boiling solvent system for recovery of acetic acid from aqueous solutions.**

(30) Priority: **07.07.83 US 511439**

(43) Date of publication of application:
**20.03.85 Bulletin 85/12**

(45) Publication of the grant of the patent:
**27.01.88 Bulletin 88/04**

(84) Designated Contracting States:
**BE DE FR GB IT**

(56) References cited:
**DE-A-2 012 674**
**US-A-2 170 834**
**US-A-2 275 862**
**US-A-2 395 010**
**US-A-4 143 066**

(73) Proprietor: **CELANESE CORPORATION**
**1211 Avenue of the Americas**
**New York New York 10036 (US)**

(72) Inventor: **Drake, Bruce D.**
**702-J, Farmhurst Dr.**
**Charlotte North Carolina (US)**
Inventor: **Howell, Carl J., Jr.**
**3944 Sussex Avenue**
**Charlotte North Carolina (US)**
Inventor: **Chen, Paul N., Sr.**
**3808 Conway Avenue**
**Charlotte North Carolina (US)**
Inventor: **Wakefield, Arch**
**3047 Point Clear Dr.**
**Charlotte North Carolina (US)**

(74) Representative: **De Minvielle-Devaux, Ian Benedict Peter et al**
**CARPMAELS & RANSFORD 43, Bloomsbury Square**
**London WC1A 2RA (GB)**

Courier Press, Leamington Spa, England.

## Description

This invention relates to a process for recovering acetic acid from moderately dilute aqueous solutions and more specifically to recovering acetic acid from 10 to 50% by weight aqueous solutions.

Separation of acetic acid from water has long been an industrially-important process. Well-known processes for achieving such a separation include the following:

2. Azeotropic dehydration distillation.
3. Liquid extraction.
    a. Low boiling solvent.
    b. High boiling solvent.

The selection of the particular separation process to be employed is largely dependent upon the concentration of acetic acid in water. For instance, at high acid concentrations above 50% by weight, simple fractionation can be considered. For intermediate concentrations, from 5 to 50%, liquid—liquid extraction is usually employed.

Liquid extraction is a means of separating two or more components of a solution. The process involves mixing the solution with an immiscible solvent, separating the two phases, and recovery of the desired materials and of solvent from the solvent phase, usually by distillation. Liquid—liquid extraction of acetic acid must always be followed by solvent-recovery systems. The selection of solvents suitable for extraction of acetic acid must be based not only on the relative affinity of the solvents for acetic acid but also on consideration of heat and other energy requirements of the extraction and solvent recovery systems.

With some solvents the extract produced has a higher ratio of acetic acid to water and, therefore, less heat is required in the subsequent distillation stage. Also, impurities in aqueous liquors which interfere with entrainment distillation processes may be removed by proper choice of a solvent for extraction. A further advantage may be obtained if the solvent used for extraction can act as an entrainer in the distillation stages.

In low boiling solvent recovery procedures, the boiling point of the solvent is below that of acetic acid. The acetic acid solution is usually contacted with a low boiling solvent in counter current fashion in conventional extraction equipment, such as a sieve tray column. Aqueous raffinate containing the small amount of acid not extracted, plus some dissolved low boiling solvent, is passed through a distillation column or raffinate stripper to remove the solvent for recycle. The solvent extract stream, containing the acid removed from the extractor feed stream, plus some dissolved water, is sent to a distillation column where the solvent and water are taken overhead. This overhead stream is phase separated so that the solvent phase can be directly recycled, and the aqueous phase is further distilled in the raffinate stripper to remove dissolved solvent. The acid exiting from the bottom of the dehydration column is passed through a final distillation step for purification, where the higher boiling contaminants are removed. The low boiling point and high mutual water solubility of the solvents commonly used for low boiling liquid extraction of acetic acid necessitates large amounts of energy for the distillation separations. As the concentration of acetic acid gets lower, the related energy costs get rapidly higher along with the capital costs associated with the large distillation hardware needed. Illustrative of low boiling point solvent recovery processes is that set forth in U.S. Patent 2,175,879.

Energy-related costs in low boiling solvent extraction techniques have been somewhat improved by the use of high boiling extractants such as the trioctylphosphine oxide extractant set forth in U.S. Patent 4,143,066. The system of the Patent, however, is only practical for recovering acetic acid in concentrations below 10% such as are found in pulp and paper waste streams.

High boiling solvent recovery systems have been extensively employed for isolating acrylic acid from aqueous crude material, such systems being set forth in U.S. Patents 3,689,541, 3,692,829, 3,781,193 and 3,657,332. Rpresentative of these patented processes is the process set forth in U.S. Patent 3,689,541 which discloses a process for isolating acrylic acid from an aqueous crude acid solution containing acrylic acid, acetic acid in a concentration of 0.5 to 8% by weight, and high boiling components boiling at a temperature higher than 223°C. The process employs a liquid—liquid extraction step, followed by multiple distillation steps. The solution is first subjected to an extraction with 3,3,5-trimethylcyclohexanone and/or isophorone as extractant, which results in the formation of an organic extract containing acrylic acid, acetic acid, high boilers and a minor proportion of water. The extract is then subjected to a first distillation to remove the high boiling components. The distillate is delivered to a second distilling column wherein a mixture of water and extractant is distilled off. The bottoms product of the second distilling step, containing acrylic acid and acetic acid, is further distilled to separate the acids; the acetic acid is distilled off and the acrylic acid remains.

As can be seen, high boiling solvent or solvent systems are available for the recovery of acrylic acid.

A series of U.S. patents to Othmer (Nos. 1 917 391, 2 050 234, 2 157 143, 2 170 834, 2 275 862 and 2 395 010) relate to recovery systems for isolating aliphatic acids from aqueous solutions thereof, for instance for isolating acetic acid from moderately dilute solutions thereof such as occur in the manufacture of acetic acid, acetic anhydride or cellulose acetate.

In particular U.S. patent No. 2 170 834 discloses a process for separating an aliphatic acid from an aqueous solution thereof by an azeotropic distillation process using a ketone or an ether as an entraining agent. Among the ketones mentioned is methyl n-amyl ketone. The patent exemplifies the use of isopropyl ether in the azeotropic distillation of a 50% aqueous formaldehyde solution.

U.S. patent No. 2 275 862 discloses a process in which an aqueous solution of an aliphatic acid is extracted with a 6—10 carbon atom ketone and the organic phase (containing extractant, acid and some water) is subjected to azeotropic distillation. The patent exemplifies the use of di-isobutyl ketone as an extractant for acetic acid.

U.S. patent No. 2 395 010 discloses a process for separating acetic acid from a moderately dilute aqueous solution which comprises subjecting the acid solution to an extraction treatment in the liquid phase with a high boiling solvent for acetic acid, to extract the acid from the aqueous solution, stripping the extract of any dissolved water in an azeotrope still and distilling the extracted acetic acid from the high boiling solvent. In one example, the patent indicates that the high boiling solvent could be methyl n-amyl ketone, which is a solvent containing a preponderant amount of a 7-carbon aliphatic ketone and having an acetic acid distribution coefficient of at least 0.32.

The process of the present invention is characterised in that the high boiling solvent contains up to 90% by weight of the 7-carbon atom aliphatic ketone and at least 10% by weight of 8- or 9-carbon aliphatic ketone or ketones.

Preferably the 7-carbon aliphatic ketone in the solvent is methyl n-amyl ketone. Preferably the 8- or 9-carbon aliphatic ketone in the solvent is selected from diisobutyl ketone, methyl hexyl ketone or ethyl amyl ketone

The process comprises as a first step feeding a weak aqueous acetic acid solution to an extractor wherein the weak acetic acid solution is contacted with the high boiling solvent system. Acetic acid is extracted from the aqueous system. The extract is then stripped of any dissolved water in an azeotrope still. The water rich phase removed in the azeotrope still is recycled back to the extractor. The extracted acetic acid is then removed from the high-boiling solvent.

The invention provides a number of advantages. It makes possible the recovery of acetic acid from moderately dilute aqueous systems by means of a high boiling solvent system, with all the attendant advantages. The invention provides a solvent system for recovering acetic acid from aqueous systems wherein the solvent meets criteria necessary for successful commercial application.

These and other advantages will become more readily apparent from the following detailed discussion of this invention.

For purposes of this invention, the term "distribution coefficient" may be defined as:

$$\frac{\text{Acetic acid concentration in the solvent phase}}{\text{Acetic acid concentration in the water phase.}}$$

The solvent system of this invention has commercial utility due to the solvent system having the following characterstics:

1) it has a high enough acid distribution coefficient for extraction,
2) it is immiscible with water for at least some acid concentrations,
3) it has a higher boiling point than acetic acid,
4) it is non-azeotropic with the acetic acid,
5) it forms a low-boiling azeotrope with water,
6) it has a low enough boiling point to allow use of steam at moderate pressure to supply energy for distillation and
7) it has a sufficient difference in specific gravity from water to provide good phase separation in the extractor.

One essential characteristic of the solvent system of the present invention is a sufficiently high acid distribution coefficient, more specifically a distribution coefficient of at least 0.32.

A low distribution coefficient has the following undesirable effects:

1. The solvent is less effective in removing acid from the extractor feed. A higher ratio of solvent to feed is required to remove the desired amount of acid from the feed. This produces extract with lower acid concentration. The extractor diameter must be increased to accommodate the increased solvent rate.

2. The azeotrope still diameter must be increased to permit larger downcomers to accommodate the increased solvent rate.

3. The main still diameter must be increased to permit larger downcomers to accommodate the increased solvent rate.

4. More energy is required to provide sensible heat for the increased solvent rate and the increased main still reflux rate, which is required because of the lower acid concentration in the main still feed.

A better understanding of the acetic acid recovery system may be had from a consideration of the drawings wherein:

Figure 1 is a graph plotting acetic acid distribution coefficient against % acetic acid in solvent phase.

Figure 2 is a flow diagram of the acetic acid route in the process of the present invention.

Figure 3 is a flow diagram of the water route in the process of the present invention.

Figure 4 is a flow diagram of the solvent route in the process of the present invention.

Figure 5 is a general flow diagram of the process of the present invention.

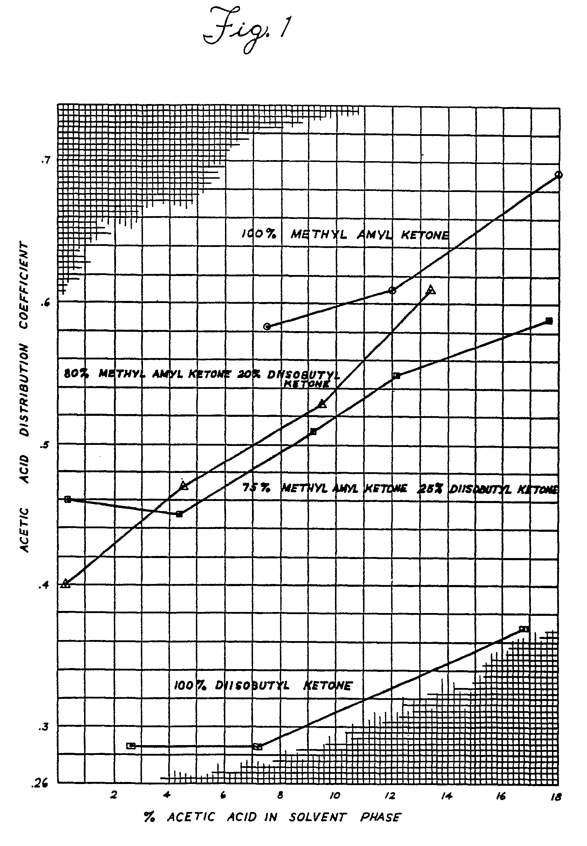

As can be seen in Figure 1 of the drawings, which is a graph plotting acetic acid in the solvent phase against acid distribution coefficient, the curve for pure methyl n-amyl ketone (a 7-carbon aliphatic ketone) has the highest distribution coefficient, while the curve for pure diisobutyl ketone (a 9-carbon aliphatic ketone) exhibits the lowest distribution coefficient. While it would appear that methyl n-amyl ketone is the most desirable solvent, methyl n-amyl ketone has a high water solubility and therefore distillation separation of the solvent is difficult. When blends of methyl n-amyl ketone and diisobutyl ketone are employed it can be seen that the blend consisting of 80% by weight methyl n-amyl ketone and 20% diisobutyl ketone exhibits for the most part the highest distribution coefficient. The blend consisting of 75% by weight methyl n-amyl ketone and 25% by weight diisobutyl ketone exhibits in general a somewhat lower distribution coefficient than the blend consisting of 80% by weight methyl n-amyl ketone and 20% by weight diisobutyl ketone.

Generally as the distribution coefficient goes up, water solubility increases in the extract. The effect for methyl n-amyl ketone and diisobutyl ketone is as follows:

| Solvent | Acid in solvent layer, wt.% | Dissolved water in solvent layer, wt.% |
|---|---|---|
| methyl n-amyl ketone | 16.85 | 5.0 |
| diisobutyl ketone | 16.85 | 3.0 |

Mixing some diisobutyl ketone with methyl n-amyl ketone allows some control of the dissolved water in the extract. Moreover, the difference in normal boiling points of methyl n-amyl ketone (150°C) and acetic acid (118°C) is 32°C. Addition of some diisobutyl ketone having a normal boiling point of 168°C to the solvent results in a solvent mixture having an increased boiling point which appears to cause distillation still separation to be easier.

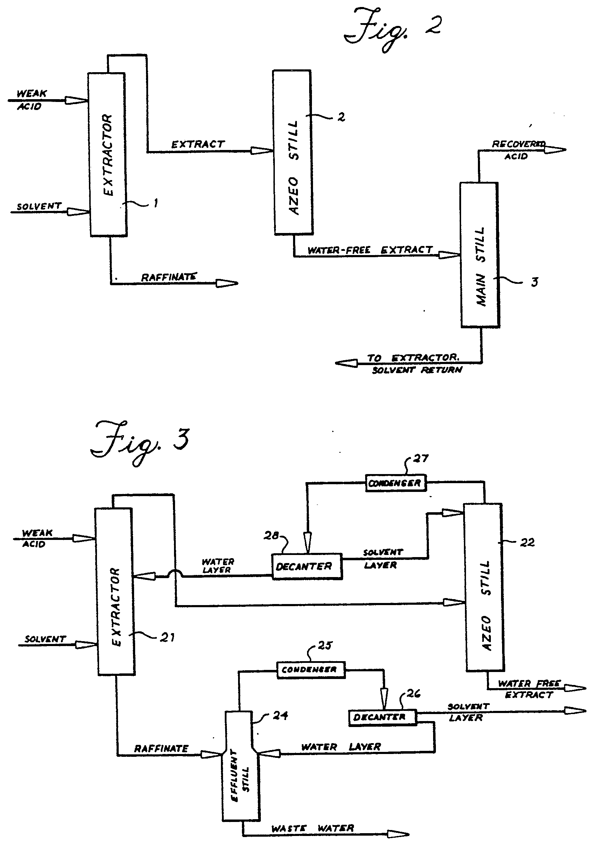

Turning to Figure 2, weak acetic acid in a concentration of about 31% by weight is fed to an extractor 1 where the weak acetic acid is contacted with a high boiling solvent. The solvent extracts the acetic acid and the extract is sent to azeotrope still 2 where it is stripped of any dissolved water. To simplify the drawing, the condenser and decanter treatment of overhead from azeotrope still 2 has not been illustrated. The bottom product is a mixture of solvent and acid which is transferred to main still 3 where distillation results in an overhead of high purity acetic acid while acid free solvent is removed at the base of main still 3.

The water route of a process of this invention may be seen in Figure 3 of the drawings. Water is fed into extractor 21 in a concentration of about 68.5% by weight of an acid water system. The majority of the feed water leaves extractor 21 as raffinate saturated with solvent. The raffinate saturated with solvent is fed into effluent still 24 wherein solvent is stripped from the raffinate, leaving solvent-free water which may be discharged as waste. The overhead from effluent still 24 is a solvent-water azeotrope which upon condensation in condenser 25 separates into a solvent and water layer in decanter 26, the solvent layer of which may be decanted. About 5% water leaves extractor 21 with the extract, and is passed on to the azeo still 22. In the azeo still 22, the water-solvent azeotrope aids the water overhead. Appreciable acid also goes overhead and the overhead on condensation in condenser 27 separates into two phases in decanter 28; the water layer which is about 15% acid is then recycled to the extractor 21.

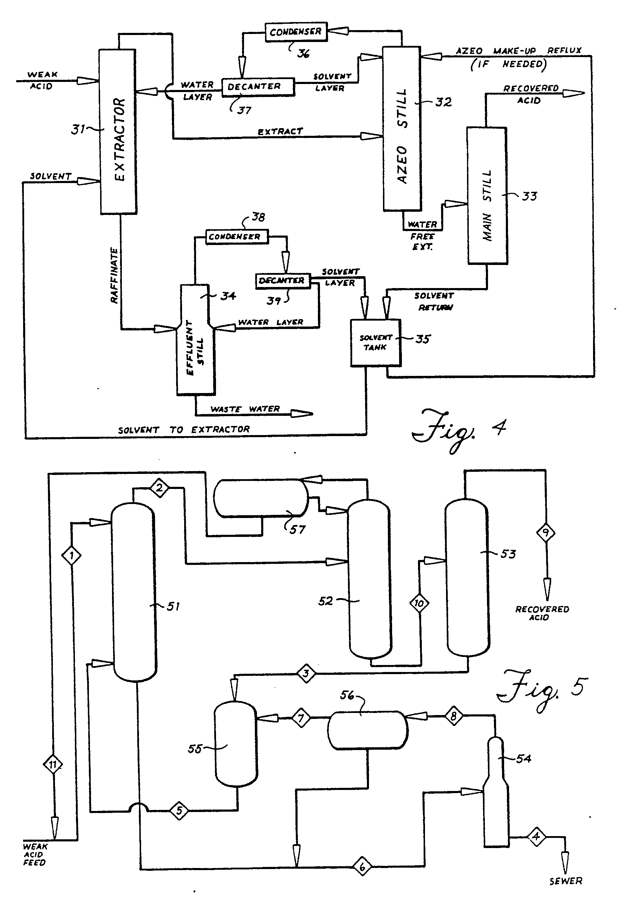

The solvent route of the process of this invention may be seen in Figure 4 of the drawings. After extracting acid from the weak acid in the extractor 31, the solvent is fed to the azeotrope still 32 as part of the extract. Any solvent which azeotropes overhead in the azeotrope still is refluxed. Overhead exiting azeotrope still 32 is passed through condenser 36 and then separated into a solvent and water layer in decanter 37, the solvent layer being returned to azeotrope still 32. If additional reflux is required, it is supplied by pumping solvent from solvent storage 35. The solvent leaving the base in the water-free extract is separated in the main still 33 as a bottom product. This recovered solvent is returned to solvent storage 35 where it is available to be recycled to extractor 31 or to azeo still 32. About 0.5% solvent is dissolved in the raffinate, leaving the base of the extractor 31. In effluent still 34, the solvent is taken overhead with azeotropic water. Upon condensation in condenser 38 two phases form in decanter 39 and the solvent phase is decanted for return to the solvent tank 35.

The following specific Examples are given for purposes of illustration and should not be considered as limiting the spirit or scope of this invention.

Example 1

Aqueous effluent from a cellulose acetate manufacturing process containing about 31% by weight of acetic acid is introduced into the upper portion of extraction tower 51 of Figure 5 of the drawings. Extraction column 51 is equipped with baffles and has a height 27.4 metres and an effective area of 0.00276 square metres. A mixture of 85% by weight methyl n-amyl ketone and 15% by weight diisobutyl ketone is introduced into the base portion of extraction tower 51 and travels upwardly therein counter-currently with respect to the aqueous solution of acetic acid. After intimate exchange of the material between the two phases, there is obtained an extract in stream 2, the acid content of which is 15.6% by weight. The extract so produced is supplied to azeotrope still 52, which is operated at about atmospheric pressure and in which the extract is freed from water, the bottoms being passed through line 10 to main still 53 where the water-free extract is separated into solvent and recovered acid, the solvent being passed to solvent tank 55. Raffinate from extraction column 51 is passed to effluent still 54 where solvent is removed from the extractor raffinate. Overhead from effluent still 54 is passed to decanter tank 56 from which the solvent layer is passed to solvent tank 55 while the water layer is discharged into the effluent still feed line. Overhead from azeo still 52 is passed to decanter tank 57 from which the solvent layer is returned as reflux to the azeo still while the water layer is returned to the weak acid feed through line 11. Weight percentages of acetic acid, water, methyl n-amyl ketone and diisobutyl ketone for each of equipment lines 1 to 11 are given in the following Table.

| Line | 1 | 2 | 3 | 4 | 5 | 6 | 7 | 8 | 9 | 10 | 11 |
|---|---|---|---|---|---|---|---|---|---|---|---|
| Wt. % acetic acid | 31.0 | 15.6 | 0.26 | | | | | | 99.82 | 16.92 | 7.2 |
| Wt. % water | 69.0 | | 0.003 | | | | 1.31 | 89.6 | 0.07 | | 92.8 |
| Wt.% methyl n-amyl ketone | | | 83.38 | 0.017 | 85.0 | 0.25 | 98.7 | 10.4 | 0.07 | | |
| Wt.% diiso-butyl ketone | | | 16.62 | nil | 15.0 | nil | | | 0.009 | | |

Comparative example

The process of Example 1 is repeated except that in place of the solvent system comprising 85% by weight methyl n-amyl ketone and 15% by weight diisobutyl ketone a solvent consisting of 100% by weight methyl n-amyl ketone is used. Due to the increased water solubility of a 100% methyl n-amyl ketone solvent, it is found that energy costs employed in operating the azeotrope still are significantly higher than energy costs employed in operating the azeotrope still in Example 1.

Example 2

The process of Example 1 is repeated except that in place of the solvent mixture of 85% by weight methyl n-amyl ketone and 15% by weight diisobutyl ketone a solvent consisting of 75% by weight methyl n-amyl ketone and 25% by weight diisobutyl ketone is employed. The solvent system of Example 2 which has a lower distribution coefficient than the solvent system of Example 1 is found to require greater amounts of energy than the process of Example 1.

**Claims**

1. A process for separating acetic acid from a moderately dilute aqueous solution which comprises subjecting the acid solution to an extraction treatment in the liquid phase with a high boiling solvent for acetic acid, being a solvent containing a preponderant amount of a 7-carbon aliphatic ketone, and having an acetic acid distribution coefficient of at least 0.32, to extract the acid from the aqueous solution, stripping the extract of any dissolved water in an azeotrope still and distilling the extracted acetic acid from the high boiling solvent, characterised in that the high boiling solvent contains up to 90% by weight of the 7-carbon aliphatic ketone and at least 10% by weight of 8- or 9-carbon aliphatic ketone or ketones.

2. The process of claim 1 wherein the 7-carbon aliphatic ketone is methyl n-amyl ketone.

3. The process of claim 1 or claim 2 wherein the remainder of the solvent is diisobutyl ketone, methyl hexyl ketone or ethyl amyl ketone.

4. The process of claim 3 wherein the high boiling solvent is a mixture of approximately 85% by weight methyl n-amyl ketone and approximately 15% by weight diisobutyl ketone.

5. The process of any one of claims 1 to 4 wherein overhead from the azeotrope still is separated into a water rich phase and a solvent rich phase, and the water rich phase is recycled back to the extraction treatment.

6. The process of any one of claims 1 to 5 comprising the steps of:

(a) contacting the aqueous solution of acetic acid with the high boiling solvent in a first column to extract the acetic acid from the water;

(b) stripping the extract of dissolved water in an azeotrope still;

(c) separating the overhead product from the azeotrope still into a water rich phase and a solvent rich phase;

(d) recycling the water rich phase from the azeotrope still back to the first column;

(e) transferring the bottom product of the azeotrope still to a main still;

(f) removing acetic acid as overhead from a distillation operation in the main still.

7. The process of claim 6 wherein the first column is maintained at a temperature of 40°C to 80°C.

8. The process of claim 6 wherein the first column is maintained at a temperature not less than 50°C and wherein the dilute aqueous solution of acetic acid is waste water from a cellulose acetate flake manufacturing process.

9. The process of any of claims 6 to 8 wherein the water rich phase contains from 3% to 25% by weight acetic acid.

**Patentansprüche**

1. Verfahren zur Abtrennung von Essigsäure aus einer mäßig verdünnten wäßrigen Lösung, bei dem die Säure-Lösung einer Extraktionsbehandlung in flüssiger Phase mit einem hochsiedenden Lösungsmittel für Essigsäure, das ein eine überwiegende Menge eines aliphatischen Ketons mit 7 Kohlenstoff-Atomen enthaltendes Lösungsmittel ist und einen Essigsäure-Verteilungskoeffizienten von wenigstens 0,32 besitzt, unterworfen wird, um die Säure aus der wäßrigen Lösung zu extrahieren, der Extrakt durch Abstreifen in einer Azeotrop-Destillationsanlage von etwa gelöstem Wasser befreit wird und die extrahierte Essigsäure aus dem hochsiedenden Lösungsmittel abdestilliert wird, dadurch gekennzeichnet, daß das hochsiedende Lösungsmittel bis zu 90 Gew.-% des aliphatischen Ketons mit 7 Kohlenstoff-Atomen und wenigstens 10 Gew.-% eines oder mehrerer aliphatischer Ketone mit 8 oder 9 Kohlenstoff-Atomen enthält.

2. Verfahren nach Anspruch 1, worin das aliphatische Keton mit 7 Kohlenstoff-Atomen Methyl-n-amylketon ist.

3. Verfahren nach Anspruch 1 oder Anspruch 2, worin der Rest des Lösungsmittels Diisobutylketon, Methylhexylketon oder Ethylamylketon ist.

4. Verfahren nach Anspruch 3, worin das hochsiedende Lösungsmittel eine Mischung aus annähernd 85 Gew.-% Methyl-n-amylketon und annähernd 15 Gew.-% Diisobutylketon ist.

5. Verfahren nach irgendeinem der Ansprüche 1 bis 4, worin aus der Azeotrop-Destillationsanlage über Kopf eine wasserreiche Phase und eine lösungsmittelreiche Phase abgetrennt werden und die wasserreiche Phase in die Extraktionsbehandlung zurückgeführt wird.

6. Verfahren nach irgendeinem der Ansprüche 1 bis 5, umfassend die Schritte des

(a) In-Berührung-Bringens der wäßrigen Lösung der Essigsäure mit dem hochsiedenden Lösungsmittel in einer ersten Kolonne, um die Essigsäure aus dem Wasser zu extrahieren;

(b) Abstreifens der gelösten Wassers aus dem Extrakt in einer Azeotrop-Destillationsanlage;

(c) Trennens des Kopfprodukts aus der Azeotrop-Destillationsanlage in eine wasserreiche Phase und eine lösungsmittelreiche Phase;

(d) Rückführens der wasserreichen Phase von der Azeotrop-Destillationsanlage zurück zu der ersten Kolonne;

(e) Überführens des Sumpfprodukts der Azeotrop-Destillationsanlage in einer Haupt-Destillationsanalage;

(f) Entfernens der Essigsäure als Kopfprodukt eines Destillationsvorgangs in der Haupt-Destillationsanlage.

7. Verfahren nach Anspruch 6, worin die erste Kolonne auf einer Temperatur von 40°C bis 80°C gehalten wird.

8. Verfahren nach Anspruch 6, worin die erste Kolonne auf einer Temperatur von nicht weniger als 50°C gehalten wird und worin die verdünnte wäßrige Lösung der Essigsäure Abwasser aus einem Herstellungsverfahren für Celluloseacetat-Flocken ist.

9. Verfahren nach irgendeinem der Ansprüche 6 bis 8, worin die wasserreiche Phase 3 bis 25 Gew.-% Essigsäure enthält.

**Revendications**

1. Procédé pour la séparation de l'acide acétique et d'une solution aqueuse modérément diluée qui consiste à soumettre la solution d'acide à un traitement d'extraction en phase liquide avec un solvant de l'acide acétique à point d'ébullition élevé, étant un solvant contenant une quantité prépondérante d'une cétone aliphatique à 7 carbones et ayant un coefficient de distribution de l'acide acétique d'au moins 0,32, pour extraire l'acide de la solution aqueuse, à séparer l'extrait de toute eau dissoute dans un four azéotrope et à distiller l'acide acétique extrait du solvant à point d'ébullition élevé, caractérisé en ce que le solvant à point d'ébullition élevé contient jusqu'à 90% en poids de la cétone aliphatique à 7 carbones et au moins 10% en poids d'une cétone ou de plusieurs cétones aliphatiques de 8 ou 9 carbones.

2. Procédé de la revendication 1, où la cétone aliphatique à 7 carbones est la méthyl n-amyl cétone.

3. Procédé de la revendication 1 ou la revendication 2, où le restant du solvant est la diisobutyl cétone, la méthyl hexyl cétone ou l'éthyl amyl cétone.

4. Procédé de la revendication 3, où le solvant à point d'ébullition élevé est un mélange d'environ 85 % en poids de méthyl n-amyl cétone et d'environ 15% en poids de diisobutyl cétone.

5. Procédé selon l'une quelconque des revendications 1 à 4, où le produit de tête du four azétrope est

6

séparé en une phase riche en eau et une phase riche en solvant, et la phase riche en eau est recyclée vers le traitement d'extraction.

6. Procédé selon l'une des revendications 1 à 5, comprenant les étapes de:

(a) mettre la solution aqueuse d'acide acétique en contact avec le solvant à point d'ébullition élevé dans une première colonne pour extraire l'acide acétique de l'eau,

(b) séparer l'extrait de l'eau dissoute dans un four azéotrope,

(c) séparer le produit de tête du four azéotrope en une phase riche en eau et une phase riche en solvant,

(d) recycler la phase riche en eau du four azéotrope vers la première colonne,

(e) transférer le produit de fond du four azéotrope à un four principal,

(f) enlever l'acide acétique en tant que produit de tête d'une opération de distillation dans le four principal.

7. Procédé de la revendication 6, où la première colonne est maintenue à une température de 40°C à 80°C.

8. Procédé de la revendication 6, où la première colonne est maintenue à une température qui n'est pas inférieure à 50°C et où la solution aqueuse diluée de l'acide acétique est l'eau résiduelle d'un procédé de fabrication de flocons d'acétate de cellulose.

9. Procédé selon l'une quelconque des revendications 6 à 8, où la phase riche en eau contient de 3% à 25% en poids d'acide acétique.

Fig. 1

*Fig. 2*

WEAK ACID → **EXTRACTOR** 1

SOLVENT → **EXTRACTOR**

EXTRACT → **AZEO STILL** 2

RAFFINATE →

WATER-FREE EXTRACT → **MAIN STILL** 3

RECOVERED ACID →

← TO EXTRACTOR. SOLVENT RETURN

*Fig. 3*

WEAK ACID → **EXTRACTOR** 21

SOLVENT → **EXTRACTOR**

WATER LAYER → **DECANTER** 28

CONDENSER 27

SOLVENT LAYER → **AZEO STILL** 22

WATER FREE EXTRACT →

SOLVENT LAYER →

CONDENSER 25

**DECANTER** 26

RAFFINATE → **EFFLUENT STILL** 24

WATER LAYER

WASTE WATER →

0 134 650

Fig. 4

Fig. 5

3